# EUROPÄISCHE PATENTANMELDUNG

(11) **EP 0 856 344 A1**
(43) Veröffentlichungstag der Anmeldung: **05.08.1998**
(21) Anmeldenummer: 98101471.5
(22) Anmeldetag: 28.01.1998
(51) Int. Cl.: B01D 9/00, B01D 9/02, C07D 233/00

(54) **Verfahren zur Reinigung von Imidazolen und imidazolbasierten Wirkstoffen durch Kristallisation**

(30) Priorität: 31.01.1997 DE 19703651
(71) Anmelder: BASF AKTIENGESELLSCHAFT, 67056 Ludwigshafen (DE)
(72) Erfinder: Fritz, Gerhard, Dr., 67125 Dannstadt-Schauernheim (DE); Wache, Harro, Dr., 67136 Fussgönheim (DE); Eck, Bernd, 68519 Viernheim (DE); Heilek, Jörg, 69245 Bammental (DE); Grünenwald, Thomas, 67158 Ellerstadt (DE); Grauenhorst, Karl Rainer, 56410 Montabaur (DE)
(74) Vertreter: Isenbruck, Günter, Dr.

(57) **Zusammenfassung**

In einem Verfahren zur Reinigung von Imidazolen und imidazolbasierten Wirkstoffen durch Kristallisation wird die Kristallisation an Kühlflächen durchgeführt, an denen Kristalle der Imidazolverbindung wachsen. Dies erfolgt vorzugsweise dadurch, daß die Kühlfläche mit einem flüssigen Stoffgemisch, das die zu reinigende Imidazolverbindung enthält, in Kontakt gebracht wird, durch Abkühlung der Kühlfläche die entsprechenden Kristalle gebildet werden, danach die nicht kristallisierte Restflüssigkeit abgeführt wird, dann die Kristalle durch Erwärmung verflüssigt werden und anschließend die an Imidazolverbindung reichere Flüssigkeit abgeführt wird.

## Beschreibung

Die vorliegende Erfindung betrifft ein Verfahren zur Reinigung von Imidazolen und imidazolbasierten Wirkstoffen durch Kristallisation.

Imidazole sind wertvolle Ausgangsstoffe für die Herstellung von Farbstoffen, Pflanzenschutzmitteln, Textilhilfsmitteln, Katalysatoren für Polyurethane und Epoxydharze, oberflächenaktiven Mitteln und Pharmazeutika, z.B. den entsprechenden Nitroimidazolen. Daneben werden Imidazole auch als Katalysatoren z.B. für Polymerisationen und Aldolkondensationen verwendet. Imidazolium-Salze haben wegen ihrer guten algiziden und bakteriziden Eigenschaften Interesse gewonnen.

Die Herstellung von Imidazolen ist wohl bekannt und z.B. in Ullmanns Encyklopädie der Technischen Chemie, Band 13, 1997, S. 173 ff. beschrieben.

Für die Aufarbeitung von Reaktionsgemischen mit dem Ziel einer Reindarstellung der Imidazole sind aus dem Stand der Technik eine Reihe von Aufarbeitungsverfahren bekannt. So sind aus EP-B-0 018 568, DE-A-3 444 337, EP-B-0 024 533 und EP-A-0 293 776 die Extraktion mit einem organischen Lösungsmittel und anschließende Destillation bekannt. Daneben ist als weiteres Trennverfahren, insbesondere für die hochreine Darstellung von Imidazolen, die Kristallisation von Imidazolen aus flüssigen Phasen bekannt. Hierbei werden Reagenzien wie z.B. Methanol, Benzol, Toluol, Diethylether im Gemisch mit Nitromethan oder Methanol zugesetzt, aus denen die Imidazole durch Kristallisation in reiner Form gewonnen werden, vgl. DD-PS 73 768, US-A-3 170 849, DE-OS 23 60 175 und der Artikel "*Synthese und Reaktionen von 1,2,4,5*-*Tetramethylimidazol; die Kristallstruktur von Pentamethylimidazolium*-*iodid*", N. Kuhn, G. Henkel und J. Kreutzberg, Z. Naturforsch. B., 46 (1991), S. 1706-1712. In dem letztgenannten Artikel wird auch die Kristallisation eines Imidazols aus einer Flüssigphase beschrieben, aus der durch Zugabe einer anorganischen Saure Imidazoliumsalzkristalle gewonnen werden. DE-OS-1 903 614 beschreibt die Abtrennung von 2-Methylimidazol in Form der freien Base in kristallinem Zustand direkt aus dem Reaktionsmedium durch Aussalzen mit anorganischen Salzen.

Beschriebener Stand der Verfahrenstechnik bei der Kristallisation von Imidazolen aus Flüssigphasen ist in allen Fällen die Massenkristallisation, bei der die Imidazole/Imidazoliumsalze als disperser Feststoff in Form einzelner Kristalle anfallen, und die Kristalle abgetrennt und gewaschen und getrocknet werden. Nachteilig bei dieser Massenkristallisation ist, daß sich zwangsläufig ein hoher Aufwand für die Isolierung der Kristalle in der gewünschten Reinheit ergibt. Die Nachteile sind im einzelnen:
- Die erforderliche Abtrennung der in der Flüssigphase suspendierten Kristalle, z.B. auf Druck-/Vakuumfiltern oder Zentrifugen,
- die zum Erreichen hoher Reinheiten erforderliche Waschung der abgetrennten Kristalle mit geeigneten Waschflüssigkeiten und
- die erforderliche Trocknung der abgetrennten und ggf. gewaschenen Kristalle.

Weiterhin nachteilig ist der Einsatz von Reagenzien, die zum Umkristallisieren für Imidazole dienen, da sie zu einem erhöhten stofflichen und verfahrenstechnischen Aufwand, z.B. Lösungsmittelaufarbeitung, führen.

Der Erfindung lag daher die Aufgabe zugrunde, ein Verfahren zur Reinigung von Imidazolverbindungen zu schaffen, das die o.g. Nachteile vermeidet und in einfacher und zuverlässiger Weise großtechnisch einsetzbar ist.

Es wurde gefunden, daß sich diese Aufgabe dadurch lösen läßt, daß die Kristallisation an Kühlflächen durchgeführt wird, an denen Kristalle der Imidazolverbindungen wachsen, und damit nicht mehr suspendiertes, bewegtes Masserkristallisat vorliegt.

Somit betrifft die Erfindung ein Verfahren zur Reinigung von Imidazolverbindungen, ausgewählt aus Imidazolen der allgemeinen Formel 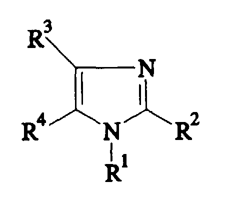 wobei R¹, R², R³ und R⁴ gleich oder verschieden sind und ein H-Atom, ein Halogenatom, eine Acetyl- oder Carbonsäuregruppe, eine verzweigte oder unverzweigte Alkylgruppe mit 1 bis 20 C-Atomen, eine Arylgruppe mit 6 bis 12 C-Atomen, eine cycloaliphatische Gruppe mit 6 bis 12 C-Atomen, eine Alkylamingruppe mit 1 bis 20 C-Atomen oder eine Nitrogruppe bedeuten, und imidazolbasierten Wirkstoffen durch Kristallisation, wobei die Kristallisation an mindestens einer Kühlfläche durchgeführt wird, an der/denen

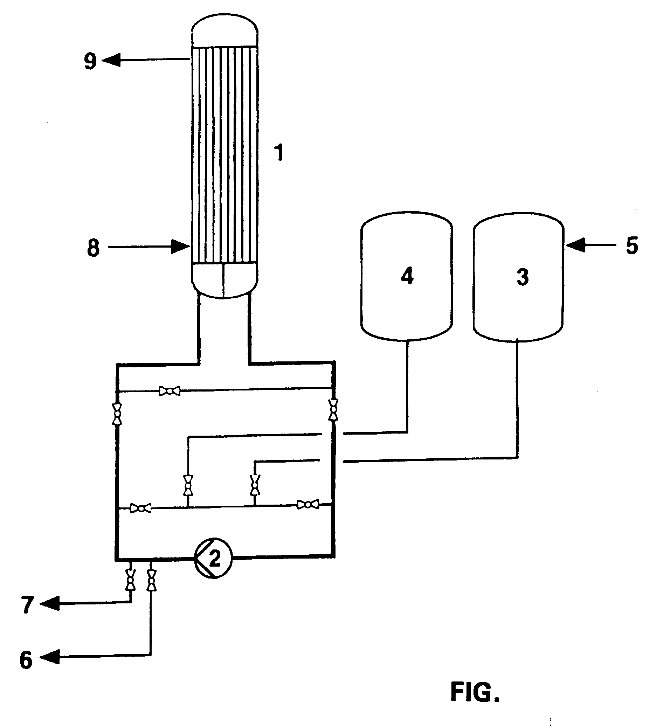

Kristalle der Imidazolverbindungen wachsen. Bevorzugte Ausführungsformen der Erfindung sind in der nachfolgenden Beschreibung, dem Ausführungsbeispiel, der Figur sowie den Unteransprüchen beschrieben.

Erfindungsgemäß geeignete Imidazole sind Verbindungen der allgemeinen Formel 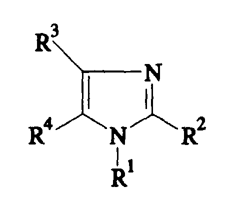 wobei R¹, R², R³ und R⁴ gleich oder verschieden sind und ein H-Atom, ein Halogenatom (F, Cl, Br, I), eine Acetyl- oder Carbonsäuregruppe, eine verzweigte oder unverzweigte Alkylgruppe mit 1 bis 20 C-Atomen, insbesondere mit 1 bis 5 C-Atomen, vorzugsweise eine Methyl- oder Ethylgruppe, eine Arylgruppe mit 6 bis 12 C-Atomen, z.B. Phenyl- oder Benzylreste, eine cycloaliphatische Gruppe mit 6 bis 12 C-Atomen, z.B. eine Cyclohexylgruppe, eine Alkylamingruppe mit 1 bis 20 C-Atomen, insbesondere mit 1 bis 5 C-Atomen, z.B. eine Methylamin- oder Ethylamingruppe oder eine Nitrogruppe darstellen. Besonders bevorzugt sind 2-Methylimidazol, 4-Methylimidazol und 2-Ethyl-4-methylimidazol. Daneben eignen sich als Imidazolverbindungen auch imidazolbasierte Wirkstoffe, ausgewählt aus den Verbindungen:
- Secnidazol, C₇H₁₁N₃O₃, α,2-Dimethyl-5-nitroimidazol-1-ethanol (systematischer Name), CAS-3366-95-8, mit der Formel 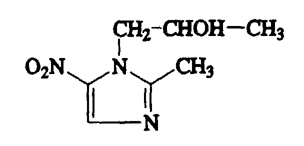
- Climbazol, C₁₅H₁₇ClN₂O₂, 1-(p-Chlorphenoxy)-1-imidazol-1-yl-3,3-dimethyl-2-butanon, CAS-38083-17-9, mit der Formel 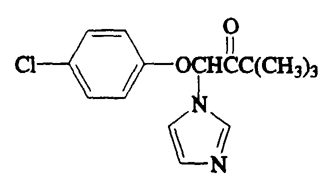
- Tinidazol, C₈H₁₃N₃ O₄S, 1-[2-(Ethylsulfonyl)ethyl]-2-methyl-5-nitroimidazol, CAS-19387-91-8, mit der Formel 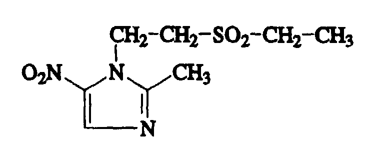
- Tioconazol, C₁₆H₁₃Cl₃N₂OS, 1-[2,4-Dichlor-β-[(2-chlor-3-thenyl)oxy]phenethyl]imidazol, CAS-65899-73-2, mit der Formel 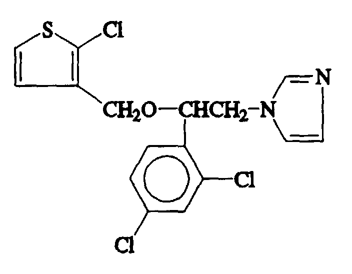
- Metronidazol, C₆H₉N₃O₃, 2-Methyl-5-nitroimidazol-1-ethanol, CAS-443-48-1, mit der Formel 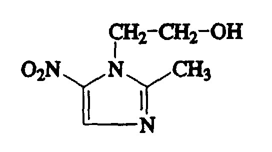
- Ketokonazol, C₂₆H₂₈Cl₂N₄O₄, (±)-cis-1-Acetyl-4-[p-[2-(2,4-dichlorphenyl)-2-(imidazol-1-ylmethyl)-1,3-dioxolan-4-yl]-methoxy]phenyl]piperazin, CAS-65277-42-1, mit der Formel 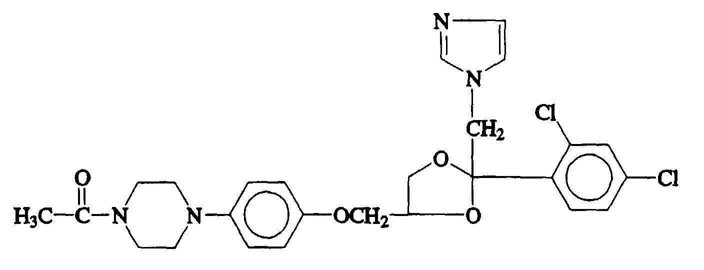
- Clotrimazol, C₂₂H₁₇ClN₂, 1-(o-Chlor-α,α-diphenylbenzyl)-imidazol, CAS-23593-75-1, mit der Formel 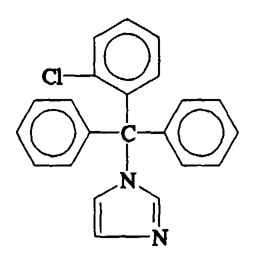
- Cimetidin, C₁₀H₁₆N₆S, 2-Cyano-1-methyl-3-[2-[[(5-methylimidazol-4-yl)methyl]thio]ethyl]-guanidin, CAS-51481-61-9, mit der Formel 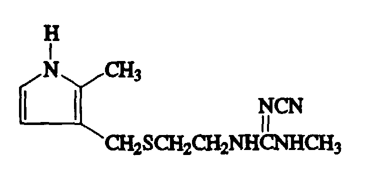
- Imazalil, C₁₄H₁₄Cl₂N₂O, 1-(b-Allyloxy-2,4-dichlorphenethyl)-imidazol, mit der Formel 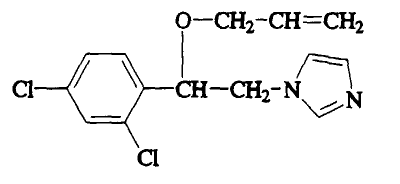 und
- Prochloraz, C₁₅H₁₆Cl₃N₃O₂, N-Propyl-N-[2-(2,4,6,-trichlorphenoxy)ethyl]imidazol-1-carboxamid, mit der Formel

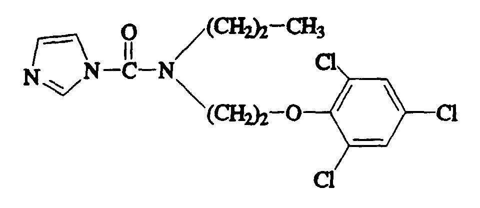

Gemäß einer vorteilhaften Weiterbildung der Erfindung werden die Kristalle der Imidazolverbindungen dadurch auf die Kühlfläche aufgebracht, daß die Kühlfläche mit einem flüssigen Stoffgemisch, das die zu reinigende Imidazolverbindung enthält, in Kontakt gebracht wird und durch Abkühlung der Kühlfläche die entsprechenden Kristalle gebildet werden. Das flüssige Stoffgemisch enthält vorzugsweise mindestens 80 Gew.-% Imidazolverbindung, insbesondere 90 bis 99,9 Gew.-% Imidazolverbindung, am meisten bevorzugt 97 bis 99,5 Gew.-% Imidazol. Der verbleibende Anteil an weiteren Komponenten unterliegt bezüglich der Art der Verunreinigungen keiner Beschränkung. Meist handelt es sich hierbei um Nebenprodukte der entsprechenden Synthesen und um Reste von Hilfsstoffen (z.B. Extraktionsmittel). Zur Bildung der Kristalle wird die Kühlfläche vorzugsweise in einem Temperaturbereich von der Lösetemperatur des jeweiligen flüssigen Stoffgemisches bis zu 80K darunter, vorzugsweise bis zu 50K darunter abgekühlt. Bei Erreichen der gewünschten Kristallmasse wird der Abkühlvorgang beendet.

Gemäß einer weiteren Ausgestaltung der Erfindung wird nach dem Aufwachsen der Kristalle die nicht kristallisierte, an Imidazolverbindung abgereicherte Restflüssigkeit abgeführt und somit von den Kühlflächen bzw. den gebildeten Kristallen entfernt. Das Abführen der Restflüssigkeit kann durch einfaches Abfließenlassen oder Abpumpen erfolgen.

Danach kann ein Wasch- und/oder Schwitzschritt durchgeführt werden. Beim Waschen werden die auf den Kühlflächen gewachsenen Kristalle mit einer Waschflüssigkeit in Kontakt gebracht und wieder von letzterer getrennt. Dadurch wird die auf den Kristallen verbleibende Restflüssigkeit durch die vorzugsweise reinere Waschflüssigkeit ausgetauscht. Insbesondere bei längerer Verweilzeit der Waschflüssigkeit auf den Kristallen erfolgt auch ein diffusiver Austausch von Verunreinigungen zwischen der reineren Waschflüssigkeit und weniger reinen Bereichen des Kristallisats. Als Waschflüssigkeit wird vorzugsweise frisches flüssiges Stoffgemisch, das die zu reinigende Imidazolverbindung enthält, oder Reinschmelze der Imidazolverbindungen verwendet. Beim Schwitzen wird nach Abführen der Restflüssigkeit die Temperatur der Kristalle auf der Kühlfläche auf einen Wert angehoben, der zwischen der Gefrierpunkttemperatur der an Imidazolverbindung abgereicherten Restflüssigkeit und der Schmelztemperatur der Rein-Imidazolverbindung liegt. Das Schwitzen ist insbesondere dann vorteilhaft, wenn die Kristalle der Imidazolverbindung nicht als kompakte Kristallschicht vorliegen, sondern als poröses, einschlußreiches Haufwerk vorliegen.

Gemäß einer weiteren Ausgestaltung der Erfindung werden danach die Kristalle durch Erwärmung verflüssigt und die entstandene, an Imidazolverbindung angereicherte Flüssigkeit abgeführt, was z.B. wiederum durch einfaches Abfließenlassen oder Abpumpen erfolgen kann. Die Verflüssigung der Kristalle erfolgt vorzugsweise in einem Temperaturbereich vom Schmelzpunkt der jeweiligen Imidazolverbindung bis 50 K darüber, insbesondere bis 30 K darüber.

In einer bevorzugten Ausführungsform der Erfindung wird auf den Kühlflächen als Restfilm nach dem Abschmelzen verbleibende, an Imidazolverbindung angereicherte Flüssigkeit als Impfkristallisat an der Kühlfläche partiell oder vollständig ausgefroren und danach erneut eine Kristallisation durchgeführt. Das Ausfrieren von Impfkristallisat kann auch dadurch erfolgen, daß vor der Kristallisation dadurch Impfkristallisat auf die Kühlfläche aufgebracht wird, indem die Kühlfläche in einem separaten Schritt mit einer, bezogen auf das zu trennende flüssige Stoffgemisch, reineren Schmelze, Lösung oder Suspension der Imidazolverbindung in Kontakt gebracht wird, anschließend davon abgetrennt wird und dann durch Abkühlen ein entsprechendes Impfkristallisat gebildet wird. Auch hierbei wird der auf den Kühlflächen verbleibende Restfilm durch Temperaturabsenkung an den Flächen partiell oder vollständig ausgefroren.

Das erfindungsgemäße Verfahren wird in einer oder mehreren Kristallisationsstufen durchgeführt. Allgemein kann man die Kristallisationsstufen in Reinigungsstufen und Abtriebsstufen einteilen. Werden die mit dem erfindungsgemäßen Verfahren kristallisierten Imidazolverbindungen nicht direkt (in einer Stufe) in der gewünschten Reinheit erhalten, können sich weitere Reinigungs-(Kristallisations-)stufen anschließen, in denen die aus der betreffenden Stufe kommenden, an Imidazolverbindung reicheren Flüssigkeiten in weiteren Reinigungsstufen aufgereinigt werden. Werden die mit dem erfindungsgemäßen Verfahren kristallisierten Imidazolverbindungen nicht in ausreichender Ausbeute erhalten (d.h., es verbleibt zu viel Imidazolverbindung in der Restflüssigkeit), kann zur Erhöhung der Ausbeute die Restflüssigkeit in weiteren Abtriebs-(Kristallisations-)stufen an Imidazolverbindung abgereichert werden. Vorzugsweise wird hierbei nach dem Gegenstromprinzip vorgegangen, d.h. die Verknüpfung der einzelnen Stufen erfolgt dergestalt, daß die an Imidazolverbindung reichere Flüssigkeit einer Stufe der nächsten Stufe zugeführt wird, und daß die an Imidazolverbindung ärmere Flüssigkeit einer Stufe der vorangehenden Stufe zugeführt wird. Die Anzahl der Kristallisationsstufen und damit auch der Reinigungs- und Abtriebsstufen hängt von der Reinigungsaufgabe ab und kann vom Fachmann im Rahmen fachüblicher Versuche ermittelt werden. Es besteht auch die Möglichkeit, auf jeder Stufe Flüssigkeitsabzüge sowohl für die an Imidazolverbindung ärmere als auch für die an Imidazolverbindung reichere Flüssigkeit vorzusehen, um diese Flüssigkeiten aus dem Verfahren auszuschleusen. Bevorzugt werden mehrere Kristallisationsstufen dadurch realisiert, daß ein Apparat für alle oder mehrere Stufen als Kristallisator dient, in dem die Stufen nacheinander gefahren werden.

Die im erfindungsgemäßen Verfahren verwendbaren Kühlflächen unterliegen an sich keiner Beschränkung und können beliebiger Form sein. Es können ein oder mehrere Kühlflächen verwendet werden. Vorzugsweise werden zylindrisch geformte Kühlflächen, z.B. Rohre, oder ebene Kühlflächen eingesetzt. Hierbei können die Kühlflächen entweder vollständig in die Flüssigkeit, aus der die Imidazolverbindung zu reinigen ist, eingetaucht sein oder nur von einem Rieselfilm dieser Flüssigkeit überströmt werden, z.B. vollständig durchströmtes oder von einem Rieselfilm durch-/überströmtes Rohr. Die Kühlflächen können auch mit einem Zulauf und einem Ablauf versehene Teile eines Wärmetauschers sein.

In einer bevorzugten Ausgestaltung der Erfindung wird während der Kristallisation das flüssige Stoffgemisch, aus dem die Imidazolverbindung auskristallisiert, an den Kühlflächen bzw. an bereits gebildeten Kristallen durch eine Zwangsströmung bewegt, oder es ruht ohne Zwangsströmung (lediglich natürliche Konvektion).

Somit läßt sich eine besonders bevorzugte Ausführungsform des erfindungsgemäßen Verfahrens als diskontinuierlicher Prozeß mit folgendem Ablauf zusammenfassen:
1. Kühlfläche(n) und das flüssige Stoffgemisch, das die zu reinigende Imidazolverbindung enthält, werden in Kontakt gebracht;
2. die Kühlflächen werden abgekühlt, so daß Kristalle der Imidazolverbindung an den Flächen wachsen;
3. der Abkühlvorgang wird beendet bei Erreichen einer gewünschten Kristallmasse;
4. die nicht kristallisierte, an Imidazolverbindung ärmere Restflüssigkeit wird von den Kühlflächen bzw. den gebildeten Kristallen entfernt;
5. die an den Kühlflächen befindlichen Kristalle werden durch Temperaturanhebung an den Flächen abgeschmolzen und die entstandene, an Imidazolverbindung angereicherte Flüssigkeit von den Flächen entfernt; und
6. auf den Flächen als Restfilm verbleibende, an Imidazolverbindung reichere Flüssigkeit kann als Impfkristallisat für den nächsten Kristallisierzyklus durch Temperaturabsenkung an den Flächen partiell oder vollständig ausgefroren werden.

Das erfindungsgemäße Verfahren ist sowohl für die Kristallisation aus der Schmelze als auch aus der Lösung anwendbar. Besonders eignet es sich zur Kristallisation aus der Schmelze.

Das erfindungsgemäße Verfahren zeichnet sich allgemein durch Einfachheit und Betriebssicherheit aus, da kein Massenkristallisat als Feststoff gehandhabt werden muß, also keine Abtrennung auf Filtern oder Zentrifugen und keine Feststofftransportvorgänge erforderlich sind.

In der Figur ist eine bevorzugte Ausführungsform der Erfindung mit zwei Reinigungsstufen dargestellt. Über die Zulaufleitung 5 wird zu reinigendes, Imidazolverbindung enthaltendes, flüssiges Gemisch in den Pufferbehälter 3 für die erste Reinigungsstufe zugeführt. Von hier aus wird das zu reinigende Gemisch mittels Pumpe 2 in den Kristallisator 1 zugeführt, der z.B. als zweizügiger Rohrbündelwärmeübertrager ausgebildet ist. Im Kristallisator 1 findet die erste Reinigungsstufe der Kristallisation statt. Die Bezugsziffern 8 und 9 bezeichnen die Zulaufleitung bzw. Ablaufleitung für das Wärmeträgermedium für den Kristallisator 1. Bei der Kristallisation werden die Kühlflächen des Kristallisators 1 mit dem zu reinigenden, flüssigen Stoffgemisch in Kontakt gebracht, anschließend werden die Kühlflächen abgekühlt, so daß Kristalle der Imidazolverbindung an den Kühlflächen wachsen. Bei Erreichen einer gewünschten Kristallmasse wird der Abkühlvorgang beendet. Die nicht kristallisierte, an Imidazolverbindung abgereicherte Restflüssigkeit wird von den Kühlflächen bzw. den gebildeten Kristallen entfernt und über die Ablaufleitung 6 abgeführt. Nun werden die an den Kühlflächen befindlichen Kristalle durch Temperaturanhebung an den Flächen abgeschmolzen und die an Imidazolverbindung angereicherte Flüssigkeit mittels Pumpe 2 in den Pufferbehälter 4 für die zweite Reinigungsstufe geführt. Auf den Kühlflächen des Kristallisators 1 verbleibende, an Imidazolverbindung reichere Flüssigkeit kann als Impfkristallisat für die zweite Reinigungsstufe durch Temperaturabsenkung ausgefroren werden. Vom Pufferbehälter 4 wird das weiter zu reinigende Stoffgemisch wiederum über Pumpe 2 in den Kristallisator 1 gepumpt. Die Kristallisation in der zweiten Reinigungsstufe verläuft analog zur ersten Reinigungsstufe. Die nach der Kristallisation vorliegende, an Imidazolverbindung abgereicherte Restflüssigkeit wird über Pumpe 2 in den Pufferbehälter 3 zurückgeführt oder gegebenenfalls wiederum über die Ablaufleitung 6 abgeführt. Die an den Kühlflächen von Kristallisator 1 befindlichen Kristalle werden durch Temperaturanhebung abgeschmolzen und über die Ablaufleitung 7 abgeführt.

Weitere Einzelheiten und Vorteile der Erfindung können dem nachfolgend beschriebenen Ausführungsbeispiel, das eine bevorzugte Ausführungsform der Erfindung darstellt, entnommen werden.

### BEISPIEL

Ein flüssiges Stoffgemisch mit einem Gehalt an 2-Methylimidazol von 99,1 Gew.-% und einem Gehalt an Verunreinigungen (Imidazol, Ethyl-methylimidazol, Methyl-vinyl-imidazol-isomere, Oxazin, Azazin und andere) von 9.000 ppm wird in einem Rührbehälter mit einer ebenen Kühlfläche kristallisiert. Die Temperierung der Kühlfläche beim Kristallisieren erfolgt in einem Temperaturbereich zwischen 143°C und 90°C. Nach Beendigung des Kristallisiervorgangs wird die Restflüssigkeit abgelassen, die an der Kühlfläche befindlichen Kristalle werden in einem Temperaturbereich zwischen 143 und 160°C abgeschmolzen und durch Ablassen der Flüssigkeit abgeführt. Das abgeschmolzene Kristallisat weist einen Gehalt an 2-Methylimidazol von 99,9 Gew.-% und einen Gehalt an Verunreinigungen von 1.000 ppm auf. Somit führt die Durchführung des erfindungsgemäßen Verfahrens zu einem hochgereinigten Imidazol.

## Patentansprüche

1. Verfahren zur Reinigung von Imidazolverbindungen, ausgewählt aus Imidazolen der allgemeinen Formel 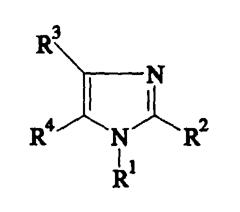 wobei R¹, R², R³ und R⁴ gleich oder verschieden sind und ein H-Atom, ein Halogenatom, eine Acetyl- oder Carbonsäuregruppe, eine verzweigte oder unverzweigte Alkylgruppe mit 1 bis 20 C-Atomen, eine Arylgruppe mit 6 bis 12 C-Atomen, eine cycloaliphatische Gruppe mit 6 bis 12 C-Atomen, eine Alkylamingruppe mit 1 bis 20 C-Atomen oder eine Nitrogruppe bedeuten,
und imidazolbasierten Wirkstoffen
durch Kristallisation,
**dadurch gekennzeichnet, daß**
die Kristallisation an mindestens einer Kühlfläche durchgeführt wird, an der Kristalle der Imidazolverbindung wachsen.

2. Verfahren nach Anspruch 1, dadurch gekennzeichnet, daß die Kristalle der Imidazolverbindung dadurch an der Kühlfläche wachsen, daß die Kühlfläche mit einem flüssigen Stoffgemisch, das die zu reinigende Imidazolverbindung enthält, in Kontakt gebracht wird und durch Abkühlung der Kühlfläche die entsprechenden Kristalle gebildet werden.

3. Verfahren nach Anspruch 2, dadurch gekennzeichnet, daß nach dem Bilden der Kristalle die nicht kristallisierte Restflüssigkeit abgeführt wird.

4. Verfahren nach Anspruch 3, dadurch gekennzeichnet, daß nach dem Abführen der Restflüssigkeit ein Wasch- und/oder Schwitzschritt mit den Kristallen durchgeführt wird.

5. Verfahren nach Anspruch 3 oder 4, dadurch gekennzeichnet, daß die Kristalle durch Erwärmung verflüssigt werden und die entstandene, an Imidazolverbindung angereicherte Flüssigkeit abgeführt wird.

6. Verfahren nach Anspruch 5, dadurch gekennzeichnet, daß auf der Kühlfläche als Restfilm verbleibende, an Imidazolverbindung angereicherte Flüssigkeit als Impfkristallisat an der Kühlfläche partiell oder vollständig ausgefroren wird und danach erneut eine Kristallisation durchgeführt wird.

7. Verfahren nach einem der Ansprüche 2 bis 5, dadurch gekennzeichnet, daß vor der Kristallisation Impfkristallisat auf die Kühlfläche aufgebracht wird, indem die Kühlfläche mit einer, bezogen auf das zu trennende flüssige Stoffgemisch, reineren Schmelze, Lösung oder Suspension der Imidazolverbindung in Kontakt gebracht wird und durch Abkühlen entsprechendes Impfkristallisat gebildet wird.

8. Verfahren nach einem der vorhergehenden Ansprüche, dadurch gekennzeichnet, daß als Kühlflächen ebene oder zylindrisch geformte Flächen eingesetzt werden.

9. Verfahren nach einem der Ansprüche 2 bis 8, dadurch gekennzeichnet, daß während der Kristallisation das flüssige Stoffgemisch ruht oder durch eine Zwangsströmung bewegt wird.

10. Verfahren nach einem der vorhergehenden Ansprüche, dadurch gekennzeichnet, daß es sich bei den imidazolbasierten Wirkstoffen um eine Verbindung handelt, ausgewählt aus:
- Secnidazol, C₇H₁₁N₃O₃, α,2-Dimethyl-5-nitroimidazol-1-ethanol (systematischer Name), CAS-3366-95-8, mit der Formel 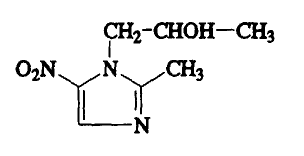
- Climbazol, C₁₅H₁₇ClN₂O₂, 1-(p-Chlorphenoxy)-1-imidazol-1-yl-3,3-dimethyl-2-butanon, CAS-38083-17-9, mit der Formel 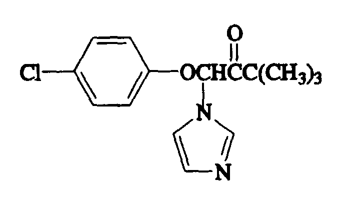
- Tinidazol, C₈H₁₃N₃O₄S, 1-[2-(Ethylsulfonyl)ethyl]-2-methyl-5-nitroimidazol, CAS-19387-91-8, mit der Formel 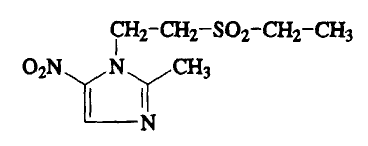
- Tioconazol, C₁₆H₁₃Cl₃N₂OS, 1-[2,4-Dichlor-β-[(2-chlor-3-thenyl)oxy]phenethyl]imidazol, CAS-65899-73-2, mit der Formel 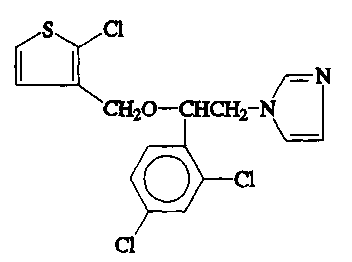
- Metronidazol, C₆H₉N₃O₃, 2-Methyl-5-nitroimidazol-1-ethanol, CAS-443-48-1, mit der Formel 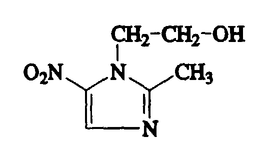
- Ketonkonazol, C₂₆H₂₈Cl₂N₄O₄, (±)-cis-1-Acetyl-4-[p-[2-(2,4-dichlorphenyl)-2-(imidazol-1-ylmethyl)-1,3-dioxolan-4-yl]-methoxy]phenyl]piperazin, CAS-65277-42-1, mit der Formel 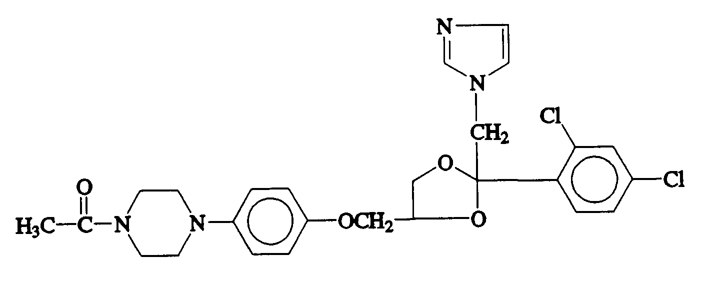
- Clotrimazol, C₂₂H₁₇ClN₂, 1-(o-Chlor-α,α-diphenylbenzyl)-imidazol, CAS-23593-75-1, mit der Formel 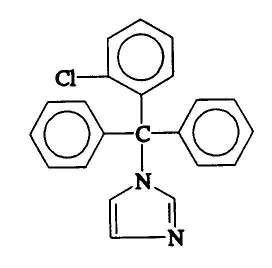
- Cimetidin, C₁₀H₁₆N₆S, 2-Cyan-1-methyl-3-[2-[[(5-methylimidazol-4-yl)methyl]thio]ethyl]-guanidin, CAS-51481-b1-9, mit der Formel 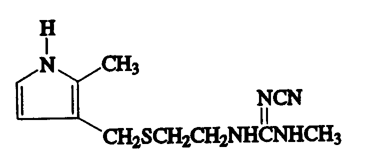
- Imazalil, C₁₄H₁₄Cl₂N₂O, 1-(b-Allyloxy-2,4-dichlorphenethyl)-imidazol, mit der Formel 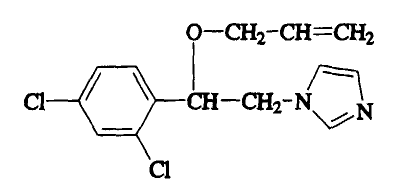 und
- Prochloraz, C₁₅H₁₆Cl₃N₃O₂, N-Propyl-N-[2-(2,4,6,-trichlorphenoxy)ethyl]imidazol-1-carboxamid, mit der Formel
